(19) Europäisches Patentamt
European Patent Office
Office européen des brevets

(11) Veröffentlichungsnummer: **0 328 984 B1**

(12) **EUROPÄISCHE PATENTSCHRIFT**

(45) Veröffentlichungstag der Patentschrift: **23.09.92**

(51) Int. Cl.$^5$: **C07D 333/32**

(21) Anmeldenummer: **89102011.7**

(22) Anmeldetag: **06.02.89**

# (54) Verfahren zur Herstellung von Thiophenethern.

(30) Priorität: **13.02.88 DE 3804522**

(43) Veröffentlichungstag der Anmeldung:
**23.08.89 Patentblatt 89/34**

(45) Bekanntmachung des Hinweises auf die Patenterteilung:
**23.09.92 Patentblatt 92/39**

(84) Benannte Vertragsstaaten:
**AT BE CH DE ES FR GB GR IT LI LU NL SE**

(56) Entgegenhaltungen:
**EP-A- 0 057 321**

**HOUBEN-WEYL: "Methoden der Organischen Chemie", Band VI/3, Teil 3, 1965, Seiten 171-173, Georg Thieme Verlag, Stuttgart, DE**

**JOURNAL OF HETEROCYCLIC CHEMISTRY, Band 14, Nr. 2, April 1977, Seiten 281-188, Hetero Corp.; S. GRONOWITZ et al.: "Inverted reactivity of aryllithium derivatives V. On the syntheses of thiocyano-, phenylsufinyl-, and phenoxy derivatives of thiophenes and furans"**

**SYNTHETIC METALS, Band 26, 1988, Seiten 153-168, Elsevier Sequoia, NL; M.R. BRYCE et al.: Synthesis and cyclic voltammetric behaviour of some 3-substituted thiophenes and pyrroles: precursors for the preparation of conducting polymers"**

**IBM J.Res.Develop. 27, 330 (1983)**

(73) Patentinhaber: **HOECHST AKTIENGESELLSCHAFT**
**Postfach 80 03 20**
**W-6230 Frankfurt am Main 80(DE)**

(72) Erfinder: **Wegener, Peter, Dr.**
**Am Eichkopf 4**
**W-6240 Königstein/Taunus(DE)**
Erfinder: **Feldhues, Michael, Dr.**
**Königsteiner Strasse 1a**
**W-6232 Bad Soden am Taunus(DE)**
Erfinder: **Litterer, Heinz, Dr.**
**Hardtstrasse 77**
**W-6208 Bad Schwalbach(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung bezieht sich auf ein vereinfachtes Verfahren zur Herstellung von Thiophenethern und die nach diesem Verfahren erhaltenen Verbindungen.

In 3-Stellung oder 3,4-Stellung substituierte Thiophene können durch Oxidation in elektrisch leitende Polymere überführt werden (IBM J. Res. Develop. 27, 330 (1983); Synthetic Metals 18 (1987), 277-282). Gegenüber dem unsubstituierten Thiophen zeichnen sich 3-Alkyl-thiophene durch ein für die Polymerisation günstigeres, weil niedrigeres Oxidationspotential, und die daraus hergestellten Polymeren durch höhere Stabilität aus.

Eine weitere Absenkung des Oxidationspotentials um ca. 0,4 V bringt die Substitution durch eine 3-Methoxy-Gruppe. Die Herstellung von 3-Methoxythiophen ist bekannt, sie erfolgt durch Umsetzung von 3-Bromthiophen mit Natriummethylat mit sehr guter Ausbeute (A. Fredga, ARKIV FÖR KEMI, Band 12 (Nr. 25)(1957). Thiophenether mit größeren Resten lassen sich jedoch auf diese Weise nicht herstellen, insbesondere nicht, wenn die zu verwendenden Alkohole noch aktive Atome oder aktive Gruppen enthalten. Jedoch wurde z.B. 3-Hexyloxythiophen durch Umsetzen von Natriumhexanolat mit Di-(3-thienyl)-iodoniumchlorid erhalten (Synthetic Metals 26 (1988), 153-168.

Andererseits ist es aus Houben-Weyl, Methoden der organischen Chemie VI/3, 172, bekannt, 3-Methoxy-2,5-diphenylfuran mit höhermolekularen Alkoholen in Gegenwart von Salzsäure umzuethern.

Es wurde nun gefunden, daß man Thiophenether mit größeren Seitengruppen leicht gewinnen kann, wenn man $C_1$-$C_3$-Alkoxythiophene mit OH-Gruppen enthaltenden Verbindungen in Gegenwart einer Säure umsetzt.

Die Erfindung betrifft somit ein Verfahren zur Herstellung von Thiophenethern der Formel I

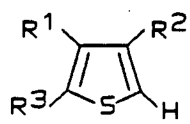

(I),

worin

$R^1$ einen geradkettigen oder verzweigten $C_3$-$C_{18}$-Alkoxyrest, einen $C_3$-$C_{18}$-Alkenyloxyrest, einen $C_3$-$C_{12}$-Alkinyloxyrest, einen $C_5$-$C_6$-Cycloalkoxyrest, einen Phenyl-$C_1$-$C_4$-alkoxyrest, einen Rest der Formel II

$-O(CH_2)_n-X$ (II),

worin n = 2 bis 6 und X ein Halogenatom, eine Hydroxyl-, Carboxylester-, $-SO_3Me$ (Me = Alkali oder $-N^+R_4^5$ mit $R^5$ = H, Alkyl), Nitro-, Cyan-, Carboxamid-, $-OCH_3$, $-OC_2H_5$ oder $-(OCH_2CH_2)_mOCH_3$-Gruppe mit m = 1 bis 3, eine quartäre Ammoniumgruppe oder eine $-P(O)(OR^4)_2$-Gruppe mit $R^4$ = H oder $C_1$-$C_4$-Alkyl ist, oder einen Rest der Glykolsäure, Thioglykolsäure, Milchsäure oder der Ester dieser Säuren bedeutet,

$R^2$ $R^1$ oder ein Wasserstoffatom, eine $C_1$-$C_{12}$-Alkylgruppe oder ein Arylrest bedeutet, und

$R^3$ ein Wasserstoffatom, eine $C_1$-$C_6$-Alkylgruppe oder eine $C_4$-$C_6$-Alkoxygruppe bedeutet, dadurch gekennzeichnet, daß man eine 3-, 2,3- oder 3,4-$C_1$-$C_3$-Alkoxythiophen zusammen mit einer eine OH-Gruppe enthaltenden Verbindung der Formel III

$R^1$-OH (III),

worin $R^1$ die obengenannte Bedeutung hat, in Gegenwart von $H_2SO_4$, $NaHSO_4$, $H_3PO_4$, polymerer Sulfonsäuren, p-Toluolsulfonsäure oder $HBF_4$ in einer Menge von 1 bis 10 mol-%, bezogen auf die Menge des Alkoxythiophens, 50 bis 300 Minuten auf eine Temperatur von 70 bis 180°C erhitzt und den entstehenden $C_1$-$C_3$-Alkohol abtrennt.

Die erfindungsgemäß hergestellten Thiophenether sind Verbindungen der Formel I

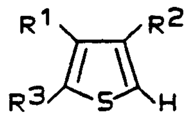

(I),

worin

$R^1$ einen geradkettigen oder verzweigten $C_3$-$C_{18}$-,vorzugsweise $C_7$-$C_{18}$-, insbesondere $C_{10}$-$C_{14}$-Alkoxyrest, einen $C_3$-$C_{18}$-, vorzugsweise $C_4$-$C_{18}$-Alkenyloxyrest, einen $C_3$-$C_{12}$-, vorzugsweise $C_3$-$C_8$-Alkinyloxyrest, einen $C_5$-$C_6$-, vorzugsweise $C_6$-Cycloalkoxyrest, einen Phenyl-$C_1$-$C_4$-alkoxyrest, einen Rest der Formel II

$-O(CH_2)_n-X$ (II),

worin n = 2 bis 6 und X ein Halogenatom, eine Hydroxyl-, Carboxylester-, $-SO_3Me$ (Me = Alkali oder $-\overset{+}{N}R_4^5$ mit $R^5$ = H, Alkyl), Nitro-, Cyan-, Carboxamid-, $-OCH_3$, $-OC_2H_5$, oder ein $-(OCH_2CH_2)_mOCH_3$-Rest mit m = 1 bis 3, eine quartäre Ammoniumgruppe oder eine $-P(O)(OR^4)_2$-Gruppe mit $R^4$ = H oder $C_1$-$C_4$-Alkyl ist, oder einen Rest der Glykolsäure, Thioglykolsäure, Milchsäure oder der Ester dieser Säuren bedeutet.

$R^2$ bedeutet $R^1$ oder ein Wasserstoffatom, eine $C_1$-$C_{12}$-, vorzugsweise $C_1$-$C_4$-Alkylgruppe oder einen Arylrest,

Vorzugsweise ist $R^2$ ein Wasserstoffatom oder eine Methylgruppe.

$R^3$ bedeutet ein Wasserstoffatom, eine $C_1$-$C_4$-Alkyl, vorzugsweise Methylgruppe, eine $C_4$-$C_6$-Alkoxygruppe

Für das erfindungsgemäße Verfahren wird ein 2-, 3-, 2,3-, 2,4-, 3,4-$C_1$-$C_3$-Alkoxythiophen eingesetzt, beispielsweise 3-Methoxythiophen, 3-Ethoxythiophen, 3-Propoxythiophen, 3-Methoxy-4-ethylthiophen, 3-Methoxy-4-butylthiophen.

Vorzugsweise werden Methoxythiophene eingesetzt.

Die $C_1$-$C_3$-Alkoxythiophene werden mit einer eine OH-Gruppe enthaltenden Verbindung der Formel (III),

$R^1$-OH (III),

worin $R^1$ die obengenannte Bedeutung hat, umgesetzt.

Beispiele für derartige Verbindungen sind geradkettige oder verzweigte $C_3$-$C_{18}$-Alkohole, $C_3$-$C_{18}$-Olefinalkohole, $C_3$-$C_{12}$-Acetylenalkohole, Cyclopentanol, Cyclohexanol, Phenylethylalkohol, Benzylalkohol, $C_2$-$C_6$-$\omega$-Halogenalkohole, Glykolsäureester, Thioglykolsäureester, Milchsäureester.

Vorzugsweise werden die vorgenannnten Alkohole eingesetzt.

Die Reaktion wird in einem inerten Lösemittel, beispielsweise einem aromatischen oder aliphatischen Kohlenwasserstoff wie Benzol, Toluol, Xylol, Cl-Benzol oder Cyclohexan durchgeführt, wobei das Lösemittel ein azeotrop siedendes Schleppmittel für den abzutrennenden Alkohol darstellen kann, oder aber im Überschuß der umzusetzenden, eine OH-Gruppe enthaltenden Verbindung. Die Umsetzung kann gegebenenfalls auch unter erhöhtem oder vermindertem Druck erfolgen. In der Regel wird die Reaktion bei einer Temperatur von 70° bis 180°C, vorzugsweise 100° bis 150°C durchgeführt. Kontinuierliches Abtrennen des frei werdenden Alkohols ist für den Reaktionsablauf vorteilhaft.

Das $C_1$-$C_3$-Alkoxythiophen und die damit umzusetzende, eine OH-Gruppe enthaltende Verbindung werden im molaren Verhältnis von 1 : 1 bis 1,5, vorzugsweise 1 : 1,1 bis 1,3 eingesetzt.

Die Reaktion wird in Gegenwart eines Katalysators durchgeführt. Die als katalysator eingesetzte Protonensäure ist $H_2SO_4$, $NaHSO_4$, $H_3PO_4$, polymere Sulfonsäure, p-Toluolsulfosäure oder $HBF_4$, und zwar im allgemeinen in katalytischen Mengen von 1 bis 10 mol-%, bezogen auf das Alkoxythiophen. Als besonders vorteilhaft hat sich das System $NaHSO_4$ oder p-Toluolsulfosäure mit Toluol als Lösemittel erwiesen.

Die Reaktion dauert 30 bis 300, vorzugsweise 50 bis 200 Minuten. In dieser Zeit ist die berechnete Menge Alkohol abgetrennt. Der saure Katalysator wird abfiltriert oder neutralisiert, das Lösemittel abgetrennt und das Produkt durch Destillation, Umkristallisation oder Chromatografie gereinigt.

Beispiele für die nach diesem Verfahren hergestellten Verbindungen sind somit:

3-Propoxythiophen, 3-Butoxythiophen, e-Pentyloxythiophen, 3-Hexyloxythiophen, 3-Heptyloxythiophen, 3-Octyloxythiophen, 3-Nonyloxythiophen, 3-Decyloxythiophen, 3-Thiodecyloxythiophen, 3-(n-Dodecyl-2-oxy)-thiophen, 3-n-Dodecyl-1-oxythiophen, 3-Tetradecyloxythiophen, 3-Pentadecyloxythiophen, 3-Hexadecyloxythiophen, 3-Octadecyloxythiophen, 3-Eicosyloxythiophen, 3-Docosyloxythiophen, 3-(2'-Ethylhexyloxy)-thiophen, 3-(2',4',4'-Trimethylpentyloxy)thiophen, 3-Cyclopentyloxythiophen, 3-Cyclohexyloxythiophen, 3-Benzyloxythiophen, 3-Propargyloxythiophen, 3-(2-Chlorethyloxy)thiophen, 3-(6-Chlorhexyloxy)thiophen, 3-(Methoxyethoxy)thiophen, 3-(Methoxyethoxyethoxy)thiophen, 3,4-Dipropoxythiophen, 3,4-Dibutoxythiophen, 3,4-Dipentyloxythiophen, 3,4-Dihexyloxythiophen, 3,4-Dioctyloxythiophen, 3,4-Dinonyloxythiophen, 3,4-Didodecyloxythiophen, 3-Methoxy-4-pentyloxythiophen, 3-Methoxy-4-hexyloxythiophen, 3-Methoxy-4-nonyloxythiophen, 3-Methoxy-4-dodecyloxythiophen, 3-Methyl-4-propargyloxythiophen, 3-Ethoxy-4-pentyloxythiophen, 3-Ethoxy-4-hexyloxythiophen, 3-Butoxy-4-dodecyloxythiophen, 3-(2'-Ethylhexyloxy)-4-methoxythiophen, 3-Propargyloxy-4-methylthiophen, 3-Butoxy-4-methylthiophen, 3-Butyl-4-methoxythiophen, 3-Dodecyl-4-methoxythiophen, 3-Dodecyl-4-butoxythiophen, 3-Butyl-4-butoxythiophen, Glykol- und Methylglykol-3,4-dioxythiophenether, 2-Butoxy-3-methoxythiophen, 2-Hexyloxy-4-methoxythiophen.

Bevorzugt hergestellt werden 3-Hexyloxythiophen, 3-Heptyloxythiophen, 3-Octyloxythiophen, 3-Nonyloxythiophen, 3-Decyloxythiophen, 3-Undecyloxythiophen, 3-Dodecyloxythiophen, 3-Tetradecyloxythiophen, 3-Pentadecyloxythiophen, 3-Hexadecyloxythiophen, 3-Octadecyloxythiophen, 3-Eicosyloxythiophen, 3-Docosyloxythiophen, 3-(2′-Ethylhexyloxy)thiophen, 3-(2′,4′,4′-Trimethylpentyloxy)thiophen, 3,4-Dihexyloxythiophen, 3,4-Diocyloxythiophen, 3,4-Dinonyloxythiophen, 3,4-Didodecyloxythiophen, 3-Methoxy-4-pentyloxythiophen, 3-Hexyloxy-4-methoxythiophen, 3-Methoxy-4-nonyloxythiophen, 3-Dodecyloxy-4-methoxythiophen, 3-Docosyloxy-4-methoxythiophen, 3-Ethoxy-4-pentyloxythiophen, 3-Ethoxy-4-hexyloxythiophen, 3-Butoxy-4-dodecyloxythiophen, 3-(2′-Ethylhexyloxy)-4-methylthiophen.

Die so erhaltenen Thiophenether können oxidativ polymerisiert werden, z.B. auf elektrochemischem Weg. Die in 2-Stellung substiuierten Thiophene können als Kettenabbrecher zur Molekulargewichtsregulierung verwendet werden.

Die Polymeren zeichnen sich in dotiertem Zustand durch gute Leitfähigkeit und Löslichkeit aus. Das Verfahren erlaubt die Herstellung einer Reihe von monomeren. Thiophenethern aus einer leicht zugänglichen Ausgangsverbindung, wobei das daraus hergestellte Polymere je nach Seitenkette in seinen Eigenschaften in weiten Grenzen variiert werden kann.

Die Polymeren können als Antistatika für Kunststoffe Verwendung finden, wobei durch Auswahl der geeigneten Seitenkette im Thiophenmonomer, bzw., -polymer eine Lösung dieses Polymers im jeweiligen Kunststoff erreicht werden kann, oder das Thiophenpolymer auf den Kunststoff abgestimmt werden kann.

Die nachfolgenden Beispiele neutral die erfindungsgemäße Reaktion.

**Beispiel 1** 3-N-Hexyloxythiophen

20 $cm^3$ 3-Methoxythiophen (0,2 mol) und 50 $cm^3$ n-Hexanol-1 wurden in 30 $cm^3$ Toluol gelöst und mit 1 g $NaHSO_4$ (0,01 mol) versetzt. Die Mischung wurde auf 125 °C erhitzt und gerührt, über eine Vigreux-Kolonne wurden etwa 10 $cm^3$ eines Gemisches von Methanol und Toluol bei 63 bis 64 °C Kopftemperatur abdestilliert, Dauer etwa 3 Stunden.Dann wurde die Mischung mit dreimal 50 $cm^3$ gesättigter $NaHCO_3$-Lösung neutral gewaschen, über $MgSO_4$ getrocknet und im Vakuum fraktioniert. Nach Abdestillieren von Toluol mit überschüssigem Hexanol gingen bei 70 °C und 0,13 mbar 34 g 3-n-Hexyloxythiophen über, entsprechend 92 % d. theor. Ausbeute. Reinheit nach GC 97 %; H-1-NMR und Massenspektrum bestätigten die Struktur.

**Beispiel 2** 3-N-Nonyloxythiophen

25 $cm^3$ 3-Methoxythiophen (0,25 mol), 50 $cm^3$ n-Nonanol-1 (0,28 mol) und 30 $cm^3$ Toluol wurden mit 1 g p-Toluolsulfosäure auf 120 °C erhitzt, im Verlauf von 3 Stunden destillierten 9,5 $cm^3$ eines Methanol-Toluol-Gemisches ab. Die Aufarbeitung erfolgte wie im Beispiel 1 beschrieben. Fraktionierung über eine 30 cm Vigreux-Kolonne ergab bei 0,26 mbar 42 g 3-n-Nonyloxythiophen mit einem Siedepunkt von 102 bis 105 °C, entsprechend 74 % d. theor. Ausbeute. H-1-NMR und Massenspektrum bestätigten die Struktur.

**Beispiel 3** 3-Dodecyloxy-4-methylthiophen

40 g 3-Brom-4-methylthiophen wurden zusammen mit 120 g Dodecanol-1 in 120 $cm^3$ Toluol gelöst und mit 2 g $NaHSO_4$ am Rückfluß zum Sieden erhitzt. Im Verlauf von 3 Stunden wurden 19 ml Methanol-Toluol-Gemisch abgetrennt. Die Lösung wurde mit Wasser neutral gewaschen, über $MgSO_4$ getrocknet und anschließend fraktioniert. Bei 141 bis 143 °C und 0,013 mbar gingen 55,6 g 3-Dodecyloxy-4-methylthiophen über. Reinheit 97 % nach GC, entsprechend 63 % d. theor. Ausbeute. Die Substanz wurde durch Massenspektrum und H-1-NMR charakterisiert.

**Beispiel 4** 3-Methoxyethoxy-4-methylthiophen

40 g 3-Methoxy-4-ethylthiophen wurden mit 60 $cm^3$ Ethylenglykol-monomethylether in 50 $cm^3$ Toluol gelöst. Unter Zusatz von 2 g $NaHSO_4$ wurde 3 Stunden am Rückfluß gekocht und 18 $cm^3$ eines Methanol-Toluol-Gemisches abgenommen.
Aufarbeitung wie in Beispiel 3. Durch fraktionierte Destillation wurden 29 g 3-Methoxyethoxy-4-methylthiophen erhalten, Kp. 62 bis 65 ° C/0,2 mbar, Reinheit 95 %, entsprechend 54 % d. Th. Ausbeute. Die Substanz wurde durch Massenspektrum und H-1-NMR charakterisiert.

**Beispiele 5 bis 13**

In der gleichen Weise wie in den angeführten Beispielen wurden die in der nachstehenden Tabelle aufgeführten Substanzen der allgemeinen Formel

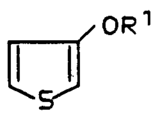

aus 3-Methoxythiophen unter Katalyse durch $NaHSO_4$ hergestellt, Siedepunkte und Ausbeuten sind angegeben, die Charakterisierung erfolgte druch H-1-NMR und Massenspektren.

| Beispiel | $R^1$ | Kp °C/mbar | Ausbeute |
|---|---|---|---|
| 5 | n-$C_{12}H_{25}$ | 140°C/0,26 | 75 % |
| 6 | n-$C_{20}H_{42}$ | Chromatogr. $CH_2Cl_2/SiO_2$ | |
| 7 | -$CH_2$-CH=$CH_2$ | 78°C/16 | 37 % |
| 8 | -CH-C≡CH | 88°C/16 | 65 % |
| 9 | -$CH_2$-CH($CH_2CH_3$)($CH_2$)$_3CH_3$ | 88°C/0,13 | 50 % |
| 10 | -Cyclohexyl | 66°C/0,06 | 38 % |
| 11 | -$CH_2$-$CH_2$-Cl | 65-68°C/0,13 | 30 % |
| 12 | -$CH_2$-$CH_2$-$OCH_3$ | 53°C/0,13 | 46 % |
| 13 | -$CH_2$-$C_6H_5$ | 99°C/0,26 | 62 % |

**Beispiel 14** 2-Hexyloxythiophen

15 $cm^3$ 2-Methoxythiophen wurden in 40 $cm^3$ n-Hexanol und 30 $cm^3$ Toluol gelöst, mit 1 g $NaHSO_4$ versetzt, einige Stunden unter Rückfluß erhitzt. Nach Abdestillieren von 6 $cm^3$ eines Azeotrops von Methanol und Toluol wurde der Rückstand mit Sodalösung gewaschen, getrocknet und fraktioniert. Es wurden 18 g 2-Hexyloxythiophen mit Kp. 66 ° C/0,26 mbar abdestilliert, Reinheit 96 %. Ausbeute 65 % d. Th.

**Beispiel 15** 3-Hexyloxy-4-butylthiophen

10 g 3-Methoxy-4-butylthiophen, hergestellt aus 3-Brom-4-butylthiophen durch Umsatz mit Natriummethylat in Methanol unter Katalyse durch CuO, Kp. 54 ° /0,26 mbar, wurden wie in Beispiel 14 in 50 $cm^3$ n-Hexanol-1 und 30 ml Toluol gelöst und mit 1 g $MeHSO_4$ versetzt. Nach Abdestillieren von 6 $cm^3$ Aezotrop wurde der Ansatz wie unter Beispiel 14 aufgearbeitet. Ausbeute 8,5 g 3-Hexyloxy-4-butylthiophen mit Kp. 112 bis 116 ° C/0,26 mbar, entsprechend 60 % d. Th.

**Beispiel 16** 3-Methoxy-4-hexyloxythiophen

10 g 3,4-Dimethoxythiophen wurden in 10 $cm^3$ n-Hexanol-1 und 30 $cm^3$ Toluol gelöst, mit 0,5 g $NaHSO_4$ versetzt und zum Rückfluß erhitzt. Es wurden 6 $cm^3$ Azeotrop abdestilliert, anschließend wurde der Ansatz, wie in Beispiel 14 aufgearbeitet. Fraktionierung ergab 3,7 g 3-Methoxy-4-hexyloxythiophen, Kp. 80 bis 85 °C/0,1 mbar. Aus dem Destillationsrückstand wurden durch Chromatographie über $SiO_2$ mit $CH_2Cl_2$ 4 g Di-hexyloxythiophen erhalten.

**Beispiel 17** 6-Chlor-hexyloxythiophen

20 $cm^3$ 3-Methoxythiophen in 30 $cm^3$ Toluol wurden mit 26 $cm^3$ 6-Chlor-1-hexanol und 1 g p-Toluolsulfonsäure versetzt und unter Rückfluß erhitzt. Es wurden 9,2 $cm^3$ eines Toluol/Methanol-Azeotrops abdestilliert. Nach Neutralisation mit wäßriger Sodalösung wurden durch Fraktionieren 14 g 6-Chlorhexyloxythiophen erhalten, Kp. 87 °C/0,01 mbar, Reinheit 98 %.

**Patentansprüche**

**1.** Verfahren zur Herstellung von Thiophenethern der Formel I

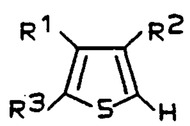

(I),

worin

$R^1$ einen geradkettigen oder verzweigten $C_3$-$C_{18}$-Alkoxyrest, einen $C_3$-$C_{18}$-Alkenyloxyrest, einen $C_3$-$C_{12}$-Alkinyloxyrest, einen $C_5$-$C_6$-Cycloalkoxyrest, einen Phenyl-$C_1$-$C_4$-alkoxyrest, einen Rest der Formel II

$-O(CH_2)_n-X$ (II),

worin n = 2 bis 6 und X ein Halogenatom, eine Hydroxyl-, Carboxylester-, $-SO_3Me$ (Me = Alkali oder $-N^+R^5_4$ mit $R^5$ = H, Alkyl), Nitro-, Cyan-, Carboxamid-, $-OCH_3$, $-OC_2H_5$ oder $-(OCH_2CH_2)_mOCH_3$-Gruppe mit m = 1 bis 3, eine quartäre Ammoniumgruppe oder eine $-P(O)(OR^4)_2$-Gruppe mit $R^4$ = H oder $C_1$-$C_4$-Alkyl ist, oder einen Rest der Glykolsäure, Thioglykolsäure, Milchsäure oder der Ester dieser Säuren bedeutet,

$R^2$ = $R^1$ oder ein Wasserstoffatom, eine $C_1$-$C_{12}$-Alkylgruppe oder einen Arylrest bedeutet, und

$R^3$ ein Wasserstoffatom, eine $C_1$-$C_6$-Alkylgruppe oder eine $C_4$-$C_6$-Alkoxygruppe bedeutet,

dadurch gekennzeichnet, daß man ein 3-, 2,3- oder 3,4-$C_1$-$C_3$-Alkoxythiophen zusammen mit einer eine OH-Gruppe enthaltenden Verbindung der Formel III

$R^1$-OH (III),

worin $R^1$ die obengenannte Bedeutung hat, in Gegenwart von $H_2SO_4$, $NaHSO_4$, $H_3PO_4$, polymerer Sulfonsäuren, p-Toluolsulfonsäure oder $HBF_4$ in einer Menge von 1 bis 10 mol-%, bezogen auf die Menge des Alkoxythiophens, 50 bis 300 Minuten auf eine Temperatur von 70 bis 180 °C erhitzt und den entstehenden $C_1$-$C_3$-Alkohol abtrennt.

**2.** Verfahren nach Anspruch 1, dadurch gekennzeichnet, daß der saure Katalysator $NaHSO_4$ oder p-Toluolsulfosäure ist.

**3.** Verfahren nach Anspruch 1, dadurch gekennzeichnet, daß der entstehende $C_1$-$C_3$-Alkohol durch azeotrope Destillation abgetrennt wird.

**4.** Verfahren nach Anspruch 1, dadurch gekennzeichnet, daß die Reaktion in Toluol als Lösemittel durchgeführt wird.

## Claims

1. A process for the preparation of thiophene ethers of the formula I

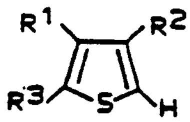

(I)

in which

$R^1$ denotes a straight-chain or branched $C_3$-$C_{18}$-alkoxy radical, a $C_3$-$C_{18}$-alkenyloxy radical, a $C_3$-$C_{12}$-alkynyloxy radical, a $C_5$-$C_6$-cycloalkoxy radical, a phenyl-$C_1$-$C_4$-alkoxy radical, a radical of the formula II

$-O(CH_2)_n-X$ (II)

in which n denotes 2 to 6 and X denotes a halogen atom, a hydroxyl, carboxylic ester, $-SO_3Me$ (Me is an alkali metal or $-\overset{+}{N}R_4^5$ where $R^5$ is H or alkyl), nitro, cyano, carboxamide, $-OCH_3$, $-OC_2H_3$ or $-(OCH_2CH_2)_mOCH_3$ group where m is 1 to 3, a quaternary ammonium group or a $-P(O)(OR^4)_2$ group where $R^4$ is H or $C_1$-$C_4$-alkyl, or denotes a radical of glycolic acid, thioglycolic acid, lactic acid or of the esters of these acids,

$R^2$ denotes $R^1$ or a hydrogen atom, a $C_1$-$C_{12}$-alkyl group or an aryl radical, and

$R^3$ denotes a hydrogen atom, a $C_1$-$C_6$-alkyl group or a $C_4$-$C_6$-alkoxy group,

which comprises heating a 3-, 2,3- or 3,4-$C_1$-$C_3$-alkoxythiophene together with a compound containing an OH group of the formula III

$R^1$-OH (III)

in which $R^1$ has the abovementioned meaning, in the presence of $H_2SO_4$, $NaHSO_4$, $H_3PO_4$, polymeric sulfonic acids, p-toluenesulfonic acid or $HBF_4$ in an amount of 1 to 10 moles %, relative to the amount of the alkoxythiophene, for 50 to 300 minutes to a temperature of 70 to 180°C and separating off the resulting $C_1$-$C_3$-alcohol.

2. The process as claimed in claim 1, wherein the acid catalyst is $NaHSO_4$ or p-toluenesulfonic acid.

3. The process as claimed in claim 1, wherein the resulting $C_1$-$C_3$-alcohol is separated off by azeotropic distillation.

4. The process as claimed in claim 1, wherein the reaction is carried out in toluene as the solvent.

## Revendications

1. Procédé pour préparer des éthers du thiophène répondant à la formule I :

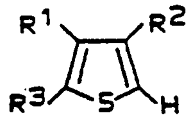

(I)

dans laquelle

$R^1$ représente un radical alcoxy en $C_3$-$C_{18}$ linéaire ou ramifié, un radical alcényloxy en $C_3$-$C_{18}$, un radical alcynyloxy en $C_3$-$C_{12}$, un radical cycloalcoxy en $C_5$ ou $C_6$, un radical phényl-$C_1$-$C_4$-alcoxy, un radical de formule II :

$-O(CH_2)_n-X$ (II)

[dans lequel n désigne un nombre de 2 à 6 et X représente un atome d'halogène, un radical hydroxy, un radical d'ester d'acide carboxylique, un radical $-SO_3Me$ (Me désignant un métal alcalin ou un radical $-\overset{+}{N}R_4^5$ , où $R^5$ représente H ou un alkyle), un radical nitro, cyano, carbamoyle, $-OCH_3$ ou $-OC_2H_5$, un radical $-(OCH_2CH_2)_mOCH_3$ dans lequel m désigne un nombre de 1 à 3, un radical ammonium quaternaire ou un radical $-P(O)(OR^4)_2$ dans lequel $R^4$ représente H ou un alkyle en $C_1$-$C_4$], ou un radical provenant de l'acide glycolique, de l'acide thioglycolique, de l'acide lactique ou d'un ester de ces acides,

$R^2$ est identique à $R^1$ ou représente un atome d'hydrogène, un alkyle en $C_1$-$C_{12}$ ou un aryle, et

$R^3$ représente un atome d'hydrogène, un alkyle en $C_1$-$C_6$ ou un alcoxy en $C_4$-$C_6$,

procédé caractérisé en ce qu'on chauffe à une température de 70 à 180 °C pendant 50 à 300 minutes un 3-, 2,3- ou 3,4-($C_1$-$C_3$-alcoxy)-thiophène avec un composé contenant un radical -OH qui répond à la formule III :

$R^1$-OH (III)

dans laquelle $R^1$ a la signification qui lui a été donnée plus haut, en présence de $H_2SO_4$, de $NaHSO_4$, de $H_3PO_4$, d'acides sulfoniques polymères, d'acide p-toluènesulfonique et de $HBF_4$ en une quantité de 1 à 10 % en moles par rapport à la quantité de l'alcoxy-thiophène, et on sépare l'alcool en $C_1$-$C_3$ qui se forme.

2. Procédé selon la revendication 1 caractérisé en ce que le catalyseur acide est $NaHSO_4$ ou l'acide p-toluènesulfonique.

3. Procédé selon la revendication 1 caractérisé en ce que l'alcool en $C_1$-$C_3$ formé est séparé par distillation azéotropique.

4. Procédé selon la revendication 1 caractérisé en ce que la réaction est effectuée dans du toluène comme solvant.